# EUROPÄISCHE PATENTSCHRIFT

(11) **EP 1 853 090 B1**
(45) Veröffentlichungstag und Bekanntmachung des Hinweises auf die Patenterteilung: **18.01.2012**
(21) Anmeldenummer: 07106550.2
(22) Anmeldetag: 20.04.2007
(51) Int. Cl.: H04R 25/00, A61B 5/12

(54) **Verfahren und Vorrichtung zum Ermitteln einer Zielverstärkungskurve für ein Hörgerät**
Method and device for determining the target gain curve of a hearing-aid
Procédé et dispositif pour déterminer la courbe de gain désirée dans une prothèse auditive

(30) Priorität: 04.05.2006 DE 102006020833
(43) Veröffentlichungstag der Anmeldung: 07.11.2007
(73) Patentinhaber: Siemens Audiologische Technik GmbH, 91058 Erlangen (DE)
(72) Erfinder: Steinbuss, Andre, 90419 Nürnberg (DE)
(74) Vertreter: Maier, Daniel Oliver

(56) Entgegenhaltungen:
- US-A- 5 197 332
- US-B1- 6 496 585
- O'NEILL G, FROSH A C, JAYARAJ S M: "Systematic errors in bone conduction audiometry." CLINICAL OTOLARYNGOLOGY, Bd. 25, Nr. 6, Dezember 2000 (2000-12), Seiten 468-470, XP002588198 ISSN: 0307-7772 DOI: 10.1046/j.1365-2273.2000.00321.x
- PEDERSEN C B, SALOMON G: "Conductive hearing loss evaluated by brief tone audiometry." ACTA OTO-LARYNGOLOGICA, Bd. 83, Nr. 5-6, Mai 1977 (1977-05), Seiten 424-428, XP008123620 ISSN: 0001-6489

## Beschreibung

Die vorliegende Erfindung betrifft ein Verfahren zum Ermitteln einer Zielverstärkungskurve für ein Hörgerät durch Messen einer Knochenleitungshörschwelle eines Patienten. Darüber hinaus betrifft die vorliegende Erfindung eine entsprechende Vorrichtung zum Ermitteln einer Zielverstärkungskurve.

Basis für die Hörgeräteanpassung sind in der Regel tonaudiometrische Daten, die durch den betreuenden Audiologen oder Hörgeräteakustiker in Form eines Tonaudiogramms erfasst werden. Diese Daten bilden die Grundlage für die Berechnung von Zielverstärkungskurven zur Kompensation des vorliegenden Hörverlustes.

In der Regel werden Knochenleitungs- und Luftleitungshörschwelle sowie die Unbehaglichkeitsschwelle tonaudiometrisch erfasst. Um den veränderten Anforderungen zur Kompensation von Schallleitungsschwerhörigkeiten oder kombinierten Schwerhörigkeiten gerecht zu werden, werden Schallleitungsanteile, d. h. die Differenz zwischen Luftleitungshörschwelle und Knochenleitungshörschwelle, deutlich stärker gewichtet und tragen, in Abhängigkeit von der gewählten Anpassstrategie, massiv zu einer veränderten Zielverstärkungskurve bei. Mögliche Fehlmessungen oder lückenhafte Messungen der Knochenleitung können daher einen massiven Einfluss auf die resultierenden Zielverstärkungskurven haben und die Spontanakzeptanz eines Hörsystems deutlich negativ beeinflussen.

Darüber hinaus ist die Messung der Knochenleitung durch ihr Wesen stark fehleranfällig. Im tieffrequenten Bereich beeinflussen Fühlschwellen das Messergebnis und im hochfrequenten Bereich hängt das Ergebnis der Messung stark von der individuellen Position des Knochenleitungshörers ab. Die Messergebnisse einer Knochenleitungsmessung sind daher wenig valide und mögliche Fehler haben einen massiven Einfluss auf die Berechnung der Zielverstärkungskurven.

Aus der Patentschrift EP 0 396 831 B1 ist ein Verfahren und eine Vorrichtung zur Bestimmung akustischer Parameter eines Hörgeräts bekannt. Dabei werden zunächst die Zielhörempfindlichkeit des Benutzers und die Übertragungsfunktion des Hörgeräts bestimmt. Anschließend wird ein Softwaremodell der Übertragungsfunktion gespeichert und die akustischen Parameter des Hörgeräts werden durch Vergleich der Hörempfindlichkeit des Softwaremodells mit der Zielhörempfindlichkeit und durch Einstellung der akustischen Parameter optimiert, um den Vergleichsfehler zu minimieren.

In der Praxis werden festgestellte Fehler der Hörgeräteeinstellung manuell korrigiert. Ist dies nicht ohne Weiteres oder rasch möglich, so wählt der Nutzer oftmals ein anderes Hörsystem.

Aus der Druckschrift US 5 197 332 A ist ein Headset zum Prüfen des Hörvermögens bekannt. Mit ihm kann die Knochenleitungshörschwelle und die Luftleitungshörschwelle gemessen werden. Für die Tests werden komprimierte Audiostimuli in einem Speichermodul gespeichert.

Die Aufgabe der vorliegenden Erfindung besteht somit darin, eine Zielverstärkungskurve für ein Hörgerät zuverlässig zu ermitteln und Messfehler gegebenenfalls zu korrigieren.

Erfindungsgemäß wird diese Aufgabe gelöst durch ein Verfahren nach Anspruch 1. Dabei bedeuten typische Schallleitungsanteile die Differenz zwischen Luftleitungshörschwelle und Knochenleitungshörschwelle für typische Hörverluste. Die Schalllmeitungsanteile charakterisieren die Mittelohrschwerhörigkeit.

Darüber hinaus wird erfindungsgemäß bereitgestellt eine Vorrichtung nach Anspruch 4.

Durch die erfindungsgemäße Korrektur der Knochenleitungsdaten vor der Berechnung der Zielverstärkungskurven lassen sich fehlerbereinigte Zielverstärkungskurven ermitteln, was sich in der Spontanakzeptanz des Hörsystems insbesondere für kombinierte Hörverluste nach der Erstanpassung niederschlägt. Für den anpassenden Audiologen beziehungsweise Hörgeräteakustiker bedeutet dies weniger Zeitaufwand für eine Nachanpassung beziehungsweise Befreiung von der Notwendigkeit eines weiteren Anpassversuchs.

Entsprechend einer erfindungsgemäßen Ausgestaltung kann derjenige Schallleitungsanteil aus der Datenbank ausgewählt werden, der zumindest in einem vorgegebenen Spektrumsabschnitt mit dem gemessenen Schallleitungsanteil am besten korreliert. Auf diese Weise können fehlerhafte Messpunkte leicht erkannt und korrigiert werden.

In vorteilhafter Weise kann das erfindungsgemäße Verfahren zur Ermittlung einer ersten Zielverstärkungskurve für eine erste Lautstärkeklasse, aber auch zur Ermittlung mindestens einer weiteren Zielverstärkungskurve für mindestens eine weitere Lautstärkeklasse für das Hörgerät verwendet werden. So lassen sich die typischen Zielverstärkungskurven für leise, mittlere und laute Pegel zuverlässig ermitteln.

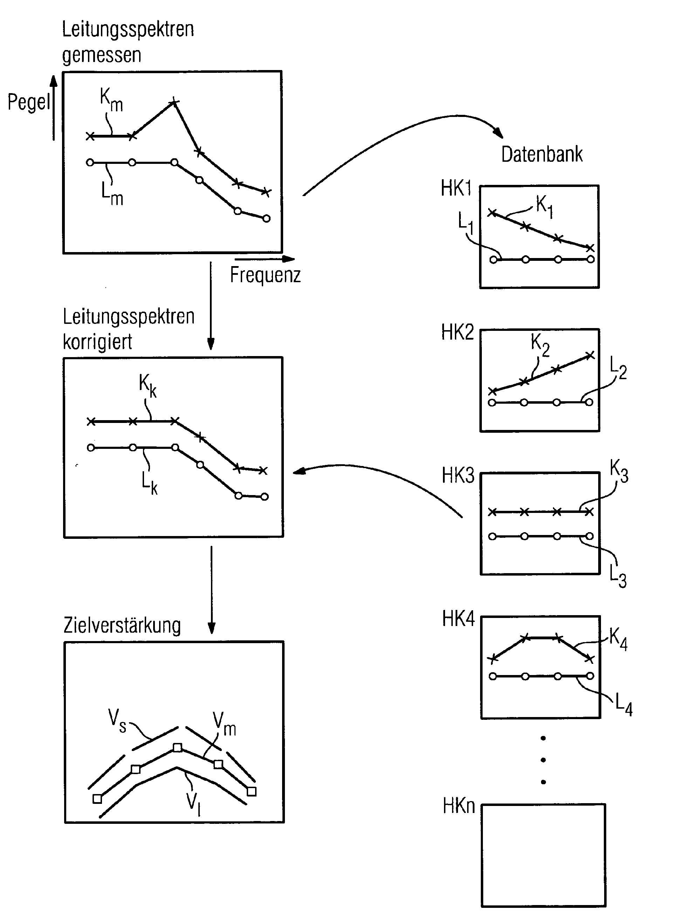

Die vorliegende Erfindung wird nun anhand der beigefügten Zeichnung näher erläutert, die ein Ablaufdiagramm zu dem erfindungsgemäßen Verfahren zeigt.

Das nachfolgend näher geschilderte Ausführungsbeispiel stellt eine bevorzugte Ausführungsform der vorliegenden Erfindung dar.

Zur Ermittlung einer korrekten Zielverstärkung beziehungsweise einer korrekten Zielverstärkungskurvenschar werden in üblicher Weise zunächst die tonaudiometrischen Daten in Form einer Knochenleitungs- und Luftleitungshörschwelle gemessen. Beide Hörschwellen geben den gerade noch wahrnehmbaren Lautstärkepegel spektral wieder. In der FIG links oben ist die gemessene Luftleitungshörschwelle Lₘ und die gemessene Knochenleitungshörschwelle Kₘ wiedergegeben. Beide Hörschwellen laufen im Wesentlichen parallel zueinander. Lediglich in einem Punkt weicht die Knochenleitungshörschwelle Kₘ davon ab, was auf eine Fehlmessung hindeutet. Um die Häufigkeit von Fehlmessungen zu reduzieren, wird erfindungsgemäß daher eine vorgeschaltete Prüfung und Korrektur der Knochenleitungswerte durchgeführt.

Zur erfindungsgemäßen Glättung oder Korrektur der Knochenleitungshörschwelle wird die Erkenntnis ausgenutzt, dass die Anzahl pathologischer Veränderungen des Mittelohrs, die zu einer Schallleitungsschwerhörigkeit führen, welche sich in der Knochenleitung niederschlagen, überschaubar ist. Darüber hinaus ist der Einfluss der Mittelohrveränderungen auf die Knochenleitungshörschwelle jeweils in Art, Grad und Verlauf weitestgehend bekannt. Daher kann auf der Basis der vorhandenen Knochenleitungswerte eine Zuordnung zu einem bekannten Krankheitsbild mit typischem Verlauf der Knochenleitungsschwelle, oder aber eine Kombination aus zweien oder mehreren Ursachen, erfolgen. Nach der Zuordnung können die Ausreißer der Knochenleitungsmessung korrigiert und mit den modifizierten Daten die Zielverstärkungskurven berechnet werden.

In dem konkreten Beispiel der FIG wurde daher eine Datenbank mit Knochenleitungs- und Luftleitungshörschwellen beziehungsweise Schalleitungsanteilen für mehrere Hörverlustsklassen, die jeweils ein spezifisches pathologisches Krankheitsbild repräsentieren, mit ihren typischen spektralen Verläufen aufgenommen. Beispielsweise nähern sich bei einer ersten Hörverlustsklasse HK1 die Knochenleitungshörschwelle K₁ und die Luftleitungshörschwelle L₁ im hochfrequenten Bereich. Bei einer zweiten symbolischen Hörverlustsklasse HK2 gehen die beiden Hörschwellen K₂ und L₂ im hochfrequenten Bereich auseinander. Bei der Hörverlustsklasse HK3 verlaufen die beiden Hörschwellen K₃ und L₃ parallel zueinander. Bei einer anderen Hörverlustsklasse HK4 liegen die beiden Hörschwellen K₄ und L₄ in einem mittleren Frequenzbereich weiter auseinander und im hohen beziehungsweise niederen Frequenzbereich nahe beisammen. Die Datenbank kann zahlreiche weitere Hörverlustsklassen HKn umfassen. Im vorliegenden Fall zeichnen sich also die Spektren der verschiedenen Klassen dadurch aus, dass sie zueinander einen speziellen Abstandsverlauf besitzen.

Die gemessenen Leitungsspektren Kₘ und Lₘ verlaufen hier mit Ausnahme eines Messpunkts im Wesentlichen parallel zueinander. Ein Vergleich mit der Datenbank ergibt, dass in der Hörverlustklasse HK3 die Knochenleitungshörschwelle K₃ mit der Luftleitungshörschwelle L₃ parallel verläuft. Die gemessenen Hörschwellen können daher mit hoher Wahrscheinlichkeit der Hörverlustsklasse HK3 zugeordnet werden. Bei dem stark abweichenden Messpunkt im gemessenen Knochenleitungsspektrum handelt es sich daher aller Wahrscheinlichkeit nach um eine Fehlmessung. Deswegen wird die gemessene Knochenleitungshörschwelle Kₘ mit Hilfe der Hörschwellen K₃, L₃ der Hörverlustsklasse HK3 korrigiert, so dass sich eine korrigierte Knochenleitungshörschwelle Kₖ ergibt. Diese korrigierte Knochenleitungshörschwelle ist in der FIG in der Mitte links dargestellt. Die zugehörige Luftleitungshörschwelle Lₖ kann unverändert sein und der gemessenen Luftleitungshörschwelle Lₘ entsprechen oder gegebenenfalls auch korrigiert sein.

Aus den korrigierten Leitungshörschwellen Kₖ und Lₖ werden die Zielverstärkungskurven Vₛ für leise Pegel, Vₘ für mittlere Pegel und V₁ für laute Pegel erstellt. Damit kann eine Hörgeräteanpassung mit Zielverstärkungskurven erfolgen, die auf korrigierten audiometrischen Daten basieren.

Bei dem oben genannten Beispiel wurden in der Datenbank ausschließlich Spektren zur Knochenleitungshörschwelle und zur Luftleitungshörschwelle beziehungsweise deren Differenzen, die Schallleitungsanteile, für jede Hörverlustsklasse aufgenommen. Alternativ könnte auch eine Datenbank ausschließlich mit Knochenleitungshörschwellen aufgebaut werden und die Korrektur nur anhand der typischen Knochenleitungshörschwellenverläufe erfolgen. Eine weitere Alternative kann darin bestehen, dass zusätzlich zu der Knochenleitungshörschwelle oder den beiden Hörschwellen für jede Hörverlustsklasse die Unbehaglichkeitsschwelle oder eine andere Schwelle aufgenommen wird. Damit lässt sich unter Umständen eine verbesserte Zuordnung zwischen den Messkurven und den Hörverlustsklassen erzielen, so dass eine zuverlässigere Korrektur von Fehlmessungen stattfinden kann.

## Patentansprüche

1. Verfahren zum Ermitteln einer Zielverstärkungskurve (Vₛ, Vₘ, V₁) für ein Hörgerät durch
- Messen einer Knochenleitungshörschwelle (Kₘ) eines Patienten,
**gekennzeichnet durch**
- Messen einer Luftleitungshörschwelle (Lₘ)
- Bereitstellen einer Datenbank mit typischen Schallleitungsanteilen für mehrere typische Hörschäden
- Auswählen eines Schallleitungsanteils aus der Datenbank anhand der gemessenen Hörschwellen (Kₘ, Lₘ)
- Glätten der gemessenen Knochenleitungshörschwelle (Kₘ) anhand des gewählten Schallleitungsanteils und
- Festlegen der Zielverstärkungskurve (Vₛ, Vₘ, V₁) in Abhängigkeit von der geglätteten Knochenleitungshörschwelle (Kₘ).

2. Verfahren nach Anspruch 1, wobei derjenige Schallleitungsanteil aus der Datenbank ausgewählt wird, der zumindest in einem vorgegebenen Spektrumsabschnitt mit der gemessenen Knochenleitungshörschwelle (Kₘ) am besten korreliert.

3. Verfahren nach einem der vorhergehenden Ansprüche, zur Ermittlung einer ersten Zielverstärkungskurve (Vₛ, Vₘ, V₁) für eine erste Lautstärkeklasse, wobei nach dem gleichen Verfahren mindestens eine weitere Zielverstärkungskurve (Vₛ, Vₘ, V₁) für mindestens eine weitere Lautstärkeklasse ermittelt wird.

4. Vorrichtung zum Ermitteln einer Zielverstärkungskurve (Vₛ, Vₘ, V₁) für ein Hörgerät mit
- einer Messeinrichtung zum Messen einer Knochenleitungshörschwelle (Kₘ) und einer Luftleitungshörschraelle (Lₘ) eines Patienten,
- einer Speichereinrichtung zum Speichern einer Datenbank mit typischen Schallleitungsanteilen für mehrere typische Hörschäden und
- einer Recheneinrichtung,
**dadurch gekennzeichnet, dass**
- die Recheneinrichtung zum automatischen Auswählen eines Schallleitungsanteils aus der Datenbank anhand der gemessenen Hörschwellen (Kₘ, Lₘ), zum Glätten der gemessenen Knochenleitungshörschwelle (Kₘ) anhand des gewählten typischen Schalleitungsanteils und zum Festlegen der Zielverstärkungskurve (Vₛ, Vₘ, V₁) in Abhängigkeit von der geglätteten Knochenleitungshörschwelle (Kₘ) ausgebildet ist.

## Claims

1. Method for determining a target amplification curve (Vₛ, Vₘ, V₁) for a hearing device by
- measuring a bone conduction hearing threshold (Kₘ) of a patient,
**characterised by**
- measuring an air conduction hearing threshold (Lₘ),
- providing a database having typical sound conduction components for a number of typical hearing impairments
- selecting a sound conduction component from the database on the basis of the measured hearing thresholds (Kₘ, Lₘ),
- smoothing the measured bone conduction hearing threshold (Kₘ) on the basis of the selected sound conduction component and
- determining the target amplification curve (Vₛ, Vₘ, V₁) as a function of the smoothed bone conduction hearing threshold (Kₘ)

2. Method according to claim 1, wherein the sound conduction component, which correlates best with the measured bone conduction hearing threshold (Kₘ) at least in a predetermined spectrum segment, is selected from the database.

3. Method according to one of the preceding claims, for determining a first target amplification curve (Vₛ, Vₘ, V₁) for a first volume category, with at least one further target amplification curve (Vₛ, Vₘ, V₁) for at least one further volume category being determined according to the same method.

4. Device for determining a target amplification curve (Vₛ, Vₘ, V₁) for a hearing device having
- a measurement facility for measuring a bone conduction hearing threshold (Kₘ) and an air conduction hearing threshold (Lₘ) of a patient,
- a storage facility for storing a database having typical sound conduction components for a number of typical hearing impairments and
- a computing facility,
**characterised in that**
- the computing facility is configured for automatically selecting a sound conduction component from the database on the basis of the measured hearing thresholds (Kₘ, Lₘ), for smoothing the measured bone conduction hearing threshold (Kₘ) on the basis of the selected typical sound conduction component and for determining the target amplification curve (Vₛ, Vₘ, V₁) as a function of the smoothed bone conduction hearing threshold (Kₘ).

## Revendications

1. Procédé de détermination d'une courbe ( Vs, Vm, Vl ) de gain visée dans une prothèse auditive par
- la mesure d'un seuil ( Km ) auditif de conduction osseuse d'un patient,
**caractérisé par**
- la mesure d'un seuil ( Lₘ ) auditif de conduction de l'air,
- la mise à disposition d'une base de données ayant des proportions typiques de conduction du son pour plusieurs troubles auditifs typiques,
- le choix d'une proportion de conduction du son dans la base de données au moyen des seuils ( Km, Lm ) auditifs mesurés,
- le lissage du seuil ( Km ) auditif de conduction osseuse mesuré au moyen de la proportion de conduction du son qui a été choisie, et
- la fixation de la courbe ( Vs, Vm, V1) de gain visée en fonction du seuil ( Km) auditif de conduction osseuse lissé.

2. Procédé suivant la revendication 1, dans lequel on choisit la proportion de conduction du son dans la base de données qui, au moins dans une partie prescrite du spectre, est le mieux en corrélation avec le seuil ( Kₘ ) auditif de conduction osseuse mesuré.

3. Procédé suivant l'une des revendications précédentes, pour la détermination d'une courbe ( Vs, Vm, Vl ) de gain visée dans une première classe de haut-parleur, dans lequel on détermine par le même procédé au moins une autre courbe ( Vs, Vm, Vl ) de gain visée pour au moins une autre classe de haut-parleur.

4. Dispositif de détermination d'une courbe ( Vs, Vm, Vl ) de gain visée d'une prothèse auditive, comprenant
- un dispositif de mesure pour mesurer un seuil ( Km ) auditif de conduction osseuse et un seuil ( Lm ) auditif de conduction de l'air d'un patient,
- un dispositif de mémoire pour mémoriser une base de données ayant des proportions typiques de conduction du son pour plusieurs troubles auditifs typiques, et
- un dispositif informatique,
**caractérisé en ce que**
- le dispositif informatique est constitué pour le choix automatique d'une proportion de conduction du son dans la base de données au moyen des seuils ( Km, Lm ) auditifs mesurés, pour lisser le seuil ( Km ) auditif de conduction osseuse mesuré au moyen de la proportion de conduction du son typique, qui a été choisie, et pour fixer la courbe ( Vs, Vm, Vl ) de gain visée en fonction du seuil ( Km ) auditif de conduction osseuse, qui a été lissé.
